# EUROPEAN PATENT APPLICATION

(11) **EP 4 342 426 A1**
(43) Date of publication of application: **27.03.2024**
(21) Application number: 23193464.7
(22) Date of filing: 25.08.2023
(51) Int. Cl.: A61F 2/24

(54) **ARTIFICIAL HEART VALVE PROSTHESIS ANCHORING DEVICE AND KIT AND USING METHOD**

(30) Priority: 31.08.2022 CN 202211061264; 31.08.2022 CN 202222342125 U
(71) Applicant: Orbusneich Medical (Shenzhen) Co., Ltd., 518038 Shenzhen (CN)
(72) Inventor: LIU, Xiyao, Shenzhen, 518038 (CN); DONG, Yuanli, Shenzhen, 518038 (CN); GUO, Lixia, Shenzhen, 518038 (CN); CAO, Yundu, Shenzhen, 518038 (CN)
(74) Representative: Cabinet Chaillot

(57) **Abstract**

The present application discloses an artificial heart valve prosthesis anchoring device and kit and a using method. The artificial heart valve prosthesis anchoring device comprises: a memory metal wire, wherein the memory metal wire is bent and encircled to form a closed loop configured to anchor an artificial heart valve stent, and the closed loop comprises: at least two clamping portions configured to form an expansion force in a radial direction and a restraint force in a circumferential direction to anchor the anchoring device between a heart valve and an adjacent blood vessel wall.

## Description

### CROSS-REFERENCE TO RELATED APPLICATION

This application claims priority to, and the benefit of, Chinese Patent Application No. 202211061264.9 filed on 31 August 2022 and Chinese Utility Model application 202222342125.5 filed on 31 August 2022. The entire contents of the foregoing application are hereby incorporated by reference for all purposes.

### TECHNICAL FIELD

The present application relates to an artificial heart valve prosthesis anchoring device and kit and a using method.

### BACKGROUND OF THE INVENTION

An aortic valve is located at the junction of a left ventricle and an aorta. When the left ventricle contracts, the aortic valve opens and blood flows into the aorta through the aortic valve. When the left ventricle relaxes, the aortic valve closes. In this case, the pressure of the aorta is greater than that of the left ventricle. A diastolic pressure is generated due to closed blood vessels and elasticity of the blood vessels. After the aortic valve is closed, the ventricle enters a diastolic phase. In this case, blood is perfused into the heart through a coronary artery. When the aortic valve is not fully closed, the left ventricle discharges blood to the aorta during systole, and the blood flows back into the left ventricle during diastole. According to the severity of aortic valve insufficiency, the amount of blood flowing backwards accounts for 10%-60% or even more of the left ventricular output. The prevalence of aortic insufficiency increases with age, and the incidence rate is as high as 2% in people of 70 years old or above. Aortic insufficiency is a chronic disease, and there is currently no approved drug treatment method.

A normal aortic root generally means a part of an ascending aorta in a pericardial cavity, and is an extension of a left ventricular outflow tract. An upper portion of the aortic root is an aortic vessel and a lower portion thereof is an aortic sinus, and the junction of the two parts is a sinus-vessel junction part. Left and right coronary arteries are important anatomical structures on the aortic valve, and the left and right coronary arteries respectively open in left and right coronary sinuses above a free edge of the valve. The height of each coronary artery opening from a valve annulus is a piece of very important data for transcatheter aortic valve replacement surgery, and the height of the coronary artery opening has a large individual variability, which is related to a body height. Autopsy abroad shows that the left coronary artery opening has a height of 12.6 mm ± 2.6 mm and the right coronary artery opening has a height of 13.2 mm ± 2.6 mm in normal people. Multi-row CT measurement shows that the left coronary artery opening has a height of 14.4 mm ± 2.9 mm and the right coronary artery opening has a height of 17.2 mm ± 3.3 mm. During design of a valve stent, it is often necessary to consider heights of the coronary artery openings, and avoid the left coronary artery opening and the right coronary artery opening to a largest extent, so as not to affect hemodynamics. It is required to design a stent as short as possible, or to increase meshes in areas of the coronary artery openings. This causes limitations to the design of the stent.

Aortic valve replacement technology requires calcification lesions in an autologous aortic valve, which can support and fix the implanted valve stent. Patients without calcification were initially thought to be incapable of undergoing transcatheter aortic valve replacement. However, in terms of the anatomical characteristics of patients with aortic valve regurgitation, native valve leaflets and aortic roots are often not necessarily accompanied by severe calcification and have certain flexibility, so that conventional transcatheter heart valve replacement cannot achieve fixation by increasing a radial supporting force. Although there are some reports on treatment of aortic regurgitation by transcatheter replacement of a heart valve based on conventional radial force support, the overall effect is not good, and the valve is prone to jumping to a second end portion of the ventricle. For this reason, how to implement a transcatheter implantation replacement device for treating aortic valve regurgitation has become an industrial problem.

In recent years, valve anchoring elements have appeared, and can help to fix a valve stent, so that patients can also undergo transcatheter aortic valve replacement. Currently, all valves with anchoring elements on the market require a transapical approach, leading to a large wound. In addition, anchoring elements in the prior art each are a memory metal material penetrating a coronary sinus to form a U-shaped structure, which uses superelasticity of the memory metal to make an anchoring device generate a radial pressure towards a central side of a blood vessel, and circumferentially clamps a valve after a valve assembly is implanted, thereby achieving an anchoring effect. In the prior art, the patent CN 101919752 B discloses an artificial valve transplantation device, as shown in FIG. 1, which is such an anchoring element D. Since a circumferentially-pressing radial force (in a direction indicated by F) in this anchoring method has a constant magnitude, and the anchoring element can only be configured to anchor a valve prosthesis stent with a specification cooperating therewith, it is necessary to configure anchoring elements with various specifications for a doctor to choose. In addition, this type of anchoring element has high requirements on model selection. If the doctor makes an improper model selection, the valve is likely to slip or fail to be completely unfolded.

### BRIEF SUMMARY OF THE INVENTION

In order to solve the problems existing in the prior art, in an aspect, the present application provides an artificial heart valve prosthesis anchoring device, comprising: a memory metal wire, wherein the memory metal wire is bent and encircled to form a closed loop configured to anchor an artificial heart valve stent, and the closed loop comprises: at least two clamping portions configured to form an expansion force in a radial direction and a restraint force in a circumferential direction to anchor the anchoring device between a heart valve and an adjacent blood vessel wall; and connecting portions that quantitatively correspond to the clamping portions, are alternately arranged with the clamping portions, and are used for transition and connection of adjacent clamping portions, wherein a curve of each clamping portion comprises at least one clamping end, and in a mounted state, each clamping end comprises: a first expansion section with a radial opening angle, and a first supporting section and a second supporting section that extend from two ends of the first expansion section, wherein at least a part of the first supporting section and the second supporting section abuts against a blood vessel wall and a valve wall, and the first expansion section abuts against a connection part between the blood vessel wall and the valve wall.

A second aspect of the present application provides an artificial aortic valve anchoring kit, comprising:
the anchoring device described above; and a claw-shaped fitting comprising a root and at least two pawls connected to the root, wherein when a coupling port of the anchoring device is coupled to the pawl, the anchoring device is in a to-be-released state; and when the coupling port of the anchoring device is uncoupled from the pawl, the anchoring device is placed at a target valve position to be self-expanded to a set state.

A third aspect of the present application provides an artificial aortic valve prosthesis anchoring device, comprising: a memory metal wire, wherein the memory metal wire is bent and encircled to form a closed loop configured to anchor an artificial heart valve stent, and the closed loop comprises: at least two clamping portions configured to form an expansion force in a radial direction and a restraint force in a circumferential direction to anchor the anchoring device between a blood vessel wall and a heart valve; and
connecting portions that quantitatively correspond to the clamping portions, are alternately arranged with the clamping portions, and are used for transition and connection of adjacent clamping portions, wherein a curve of each clamping portion comprises two clamping ends adjacent and connected to each other, and in a mounted state, each clamping end comprises: a first expansion section with a radial opening angle, the first expansion section being generally V-shaped or U-shaped; and a first supporting section and a second supporting section that extend from two ends of the first expansion section; when the anchoring device is applied to an anchoring position, the first expansion section and the extended first supporting section comprise at least a part in continuous contact with the heart valve or form multipoint contact with the heart valve; and the first expansion section and the extended second supporting section comprise at least a part in continuous contact with the blood vessel wall or form multipoint contact with the blood vessel wall.

A fourth aspect of the present application provides a using method for an artificial aortic valve prosthesis anchoring device, comprising: self-expanding, after the anchoring device according to any one of the embodiments is placed at an anchoring position by means of a catheter, the anchoring device to release and form an expansion force towards an artificial aortic valve stent and a blood vessel wall, and subjecting the anchoring device to an opposite acting force from the artificial aortic valve stent and the blood vessel wall, thereby forming a force balance to anchor the artificial aortic valve stent.

### BRIEF DESCRIPTION OF THE DRAWINGS

The features and advantages of the present application may be further understood with reference to the remaining parts of this description and the accompanying drawings. Identical assemblies in these accompanying drawings are numbered with the same reference numerals. In some cases, a sub-marker is shown after a reference numeral and hyphen to represent one of many similar assemblies. Any one mentioned reference numeral with an existing sub-marker not specifically identified refers to all such similar assemblies.

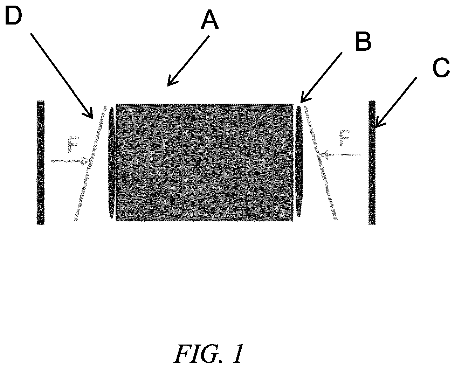

FIG. 1 is a schematic diagram of force application of an anchoring element in the prior art in a mounted state;
FIG. 2 is a schematic diagram of an anchoring device according to one of embodiments of the present application in a mounted state;
FIG. 3 is a schematic diagram of force application of an anchoring device according to the present application in a mounted state;
FIG. 4 is a schematic structure diagram of an anchoring device according to one of embodiments of the present application;
FIG. 5 is a schematic structure diagram of a clamping end according to the embodiment of the present application shown in FIG. 4;
FIG. 6 is a schematic structure diagram of a clamping end according to another embodiment of the present application;
FIG. 7 is a schematic structure diagram of a clamping end according to still another embodiment of the present application;
FIG. 8A is a schematic diagram of a state in which an anchoring device according to one of embodiments of the present application is anchored to a smaller heart valve prosthesis;
FIG. 8B is a schematic diagram of a state in which an anchoring device according to one of embodiments of the present application is anchored to a larger heart valve prosthesis;
FIG. 9 is a schematic structure diagram of an anchoring device according to another embodiment of the present application;
FIG. 10 is a schematic structure diagram of a clamping end according to the embodiment shown in FIG. 9;
FIG. 11 is a schematic structure diagram of an anchoring device according to still another embodiment of the present application;
FIG. 12 is a schematic diagram of one of anchoring device kits according to the present application;
FIG. 13 is a schematic diagram of another anchoring device kit according to the present application;
FIG. 14 is a schematic local state diagram of a claw-shaped fitting according to the present application when combined with an anchoring device; and
FIG. 15 is a schematic structure diagram of one of anchoring devices according to the present application.

### DETAILED DESCRIPTION OF EMBODIMENTS

The embodiments are described in greater detail below with reference to the following examples, which are provided herein by way of illustration only and are not intended to be limiting.

The present application has many variations which can be expected by those skilled in the art, and the use effect of the present application can be achieved.

In the present application, the term "comprising" means including, but not limited to, the following elements, that is, not excluding other elements.

In the present application, the terms "about" and "approximately" refer to accuracy intervals that would be understood by those skilled in the art so as to still ensure the technical effects of the features discussed. The terms generally denote a deviation of 10%, preferably 5%, from the indicated value.

In the present application, an artificial heart valve prosthesis is a medical product implanted in a heart to function instead of a natural heart valve in the human heart, and generally includes a stent and artificial valves. The stent is cylindrical, and the valves (two or three) are arranged in the stent to form a valve shape.

In the present application, an artificial aortic valve prosthesis anchoring device is a medical product implanted in an aortic root to function instead of a natural aortic valve, and generally includes a stent and artificial valves. The stent is cylindrical, and the valves are arranged in the stent to form a valve shape.

In the present application, a radial direction means a direction the same as a diameter direction of the stent of the artificial heart valve prosthesis implanted in a human body as a reference, and includes a direction towards a center of a circle and a direction opposite to the center of the circle.

In the present application, a circumferential direction means a direction along a circumferential surface of a stent cylinder with the stent of the artificial heart valve prosthesis implanted in the human body as the reference.

In the present application, a memory metal includes, but is not limited to, nickel-titanium, titanium-nickel-copper, titanium-nickel-iron, titanium-nickel-chromium, copper-nickel alloys, copper-aluminum alloys, copper-zinc alloys, and iron alloys (Fe-Mn-Si, and Fe-Pd), and may have a diameter of 0.1-3 mm.

In the present application, an upper position at which the anchoring device is anchored to a heart valve prosthesis is taken as a reference position, and one end thereof away from the heart is used as an upper end or a high end, while the other end thereof close to the heart is used as a lower end or a bottom end; and one side close to the heart valve is used as the inside, and the other side close to a blood vessel wall is used as the outside.

The present application relates to an anchoring device for a heart valve implanted in a transcatheter manner, and in particular, to an anchoring device for a heart valve prosthesis implanted in a transcatheter manner, which has an adaptive function and can adapt to various styles and specifications. The anchoring device includes two or more layers of memory metal structures with interacting forces in a single coronary sinus, which can achieve mutual compensation of acting forces. A side next to the valve can generate a pressure toward a center of a blood vessel, and a side next to the aortic root can generate a pressure towards the blood vessel wall. After the artificial valve is implanted, the two forces squeeze each other, so that the anchoring device is deformed. In addition, a part of a radial force pointing to the blood vessel wall is transmitted to the blood vessel wall. In this case, a combination of the anchoring device and the valve stent can be fixed to the aortic root without displacement, and the anchoring device can also be self-adapted to the shape of the valve stent, so as to adapt to heart valve prostheses with various specifications and appearances.

In a first aspect, the present application provides an artificial heart valve prosthesis anchoring device, comprising: a memory metal wire, wherein the memory metal wire is bent and encircled to form a closed loop configured to anchor an artificial heart valve stent, and the closed loop comprises: at least two clamping portions configured to form an expansion force in a radial direction and a restraint force in a circumferential direction to anchor the anchoring device between a heart valve and an adjacent blood vessel wall; and connecting portions that quantitatively correspond to the clamping portions, are alternately arranged with the clamping portions, and are used for transition and connection of adjacent clamping portions, wherein a curve of each clamping portion comprises at least one clamping end, and in a mounted state, each clamping end comprises: a first expansion section with a radial opening angle, and a first supporting section and a second supporting section that extend from two ends of the first expansion section, wherein at least a part of the first supporting section and the second supporting section abuts against a blood vessel wall and a valve wall, and the first expansion section abuts against a connection part between the blood vessel wall and the valve wall. The expansion section with the radial opening angle makes the anchoring device form bidirectional (towards a blood vessel wall side and a heart valve side) expansion forces, and the first supporting section and the second supporting section form enough longitudinal support to make the anchoring more stable and adapt to heart valve prostheses with different sizes.

In at least one embodiment, each of the connecting portions is a curved section between two adjacent clamping portions, and a middle portion of the connecting portion is provided with a coupling port configured to be coupled to or released from an instrument for operating the anchoring device. In at least one embodiment, the coupling port is ring-shaped or hook-shaped.

In at least one embodiment, when the anchoring device is applied to an anchoring position, the second supporting section comprises at least a part in continuous contact with the blood vessel wall. In at least one embodiment, when the anchoring device is applied to an anchoring position, the second supporting section forms multipoint contact with the blood vessel wall. In at least one embodiment, when the anchoring device is applied to an anchoring position, the first supporting section comprises at least a part in continuous contact with the heart valve. In at least one embodiment, when the anchoring device is applied to an anchoring position, the first supporting section is in contact with the heart valve to form multipoint contact.

In at least one embodiment, the clamping end further comprises a second expansion section with a second radial opening angle, the second expansion section is connected to the first expansion section by the second supporting section, and a third supporting section extends from the other end of the second expansion section. The expansion sections with a plurality of radial opening angles enable the anchoring device to achieve a stronger bidirectional expansion effect (towards the blood vessel wall side and the heart valve side).

In at least one embodiment, the second expansion section and the first expansion section are rotationally symmetric. In at least one embodiment, the first expansion section and/or the second expansion section are/is generally V-shaped or U-shaped. In at least one embodiment, the second supporting section connecting the first expansion section to the second expansion section forms contact with the blood vessel wall and/or the heart valve. In at least one embodiment, when the anchoring device is applied to an anchoring position, the third supporting section forms continuous-part contact or multipoint contact with the blood vessel wall.

In at least one embodiment, the clamping portion of the anchoring device comprises two substantially symmetrical clamping ends. In at least one embodiment, the two clamping ends of the anchoring device are connected by a fourth supporting section, and the fourth supporting section is a curved section arranged in the circumferential direction.

In at least one embodiment, a second memory metal wire is further wound around the anchoring device, and the second memory metal wire is wound at least around a part of the clamping portion.

A second aspect of the present application provides an artificial aortic valve anchoring kit, comprising:
the anchoring device according to any one of the embodiments; and a claw-shaped fitting comprising a root and at least two pawls connected to the root, wherein when a coupling port of the anchoring device is coupled to the pawl, the anchoring device is in a to-be-released state; and when the coupling port of the anchoring device is uncoupled from the pawl, the anchoring device is placed at a target valve position to be self-expanded to a set state.

In at least one embodiment, the kit further comprises a manipulator, wherein an end portion of the manipulator is connected to the claw-shaped fitting, and the manipulator is configured to push the anchoring device and the claw-shaped fitting towards a distal port of a catheter by means of the catheter until the anchoring device and the claw-shaped fitting are pushed out of the distal port of the catheter.

A third aspect of the present application provides an artificial aortic valve prosthesis anchoring device, comprising: a memory metal wire, wherein the memory metal wire is bent and encircled to form a closed loop configured to anchor an artificial heart valve stent, and the closed loop comprises: at least two clamping portions configured to form an expansion force in a radial direction and a restraint force in a circumferential direction to anchor the anchoring device between a blood vessel wall and a heart valve; and connecting portions that quantitatively correspond to the clamping portions, are alternately arranged with the clamping portions, and are used for transition and connection of adjacent clamping portions, wherein a curve of each clamping portion comprises two clamping ends adjacent and connected to each other, and in a mounted state, each clamping end comprises: a first expansion section with a radial opening angle, the first expansion section being generally V-shaped or U-shaped; and a first supporting section and a second supporting section that extend from two ends of the first expansion section; when the anchoring device is applied to an anchoring position, the first expansion section and the extended first supporting section comprise at least a part in continuous contact with the heart valve or form multipoint contact with the heart valve; and the first expansion section and the extended second supporting section comprise at least a part in continuous contact with the blood vessel wall or form multipoint contact with the blood vessel wall.

In at least one embodiment, the clamping end further comprises a second expansion section with a second radial opening angle, the second expansion section is connected to the first expansion section by the second supporting section, and a third supporting section extends from the other end of the second expansion section. In at least one embodiment, a second memory metal wire is further wound around the anchoring device, and the second memory metal wire is wound at least around a part of the clamping portion.

A fourth aspect of the present application provides a using method for an artificial aortic valve prosthesis anchoring device, comprising: self-expanding, after the anchoring device according to any one of the embodiments is placed at an anchoring position by means of a catheter, the anchoring device to release and form an expansion force towards an artificial aortic valve stent and a blood vessel wall, and subjecting the anchoring device to an opposite acting force from the artificial aortic valve stent and the blood vessel wall, thereby forming a force balance to anchor the artificial aortic valve stent.

In at least one embodiment, the anchoring device is applied to the anchoring position by means of a manipulator and a claw-shaped fitting via an interventional catheter. In at least one embodiment, the anchoring position is an aortic sinus.

The structure and implementations of the present application will be described in more detail below with reference to the accompanying drawings.

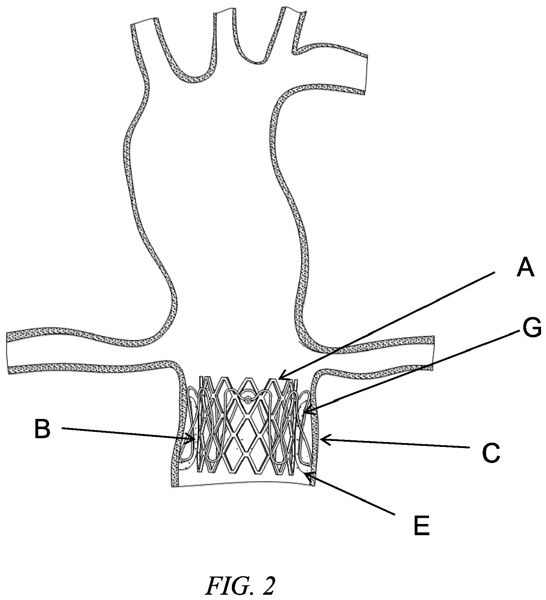

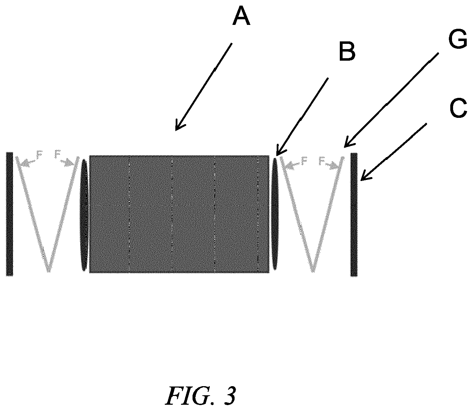

FIGS. 2 and 3 show schematic diagrams of mounting an anchoring device G according to the present application to an aortic sinus E, with an outer side being an aortic vessel wall C and an inner side being a heart valve B. An artificial heart valve prosthesis A is arranged on the inner side of the heart valve B. The anchoring device G applies forces (in a direction indicated by an arrow F) towards the heart valve B (that is, in the direction of the valve prosthesis A) and the vessel wall C (that is, the aortic vessel wall). Correspondingly, under the action of reaction forces, the anchoring device G achieves the balance of forces in the aortic sinus, so that the artificial heart valve prosthesis A is firmly anchored, the artificial heart valve prosthesis A is not likely to shift in the body, and the anchoring device can also take the anchoring of artificial heart valve prostheses A with various specifications into account.

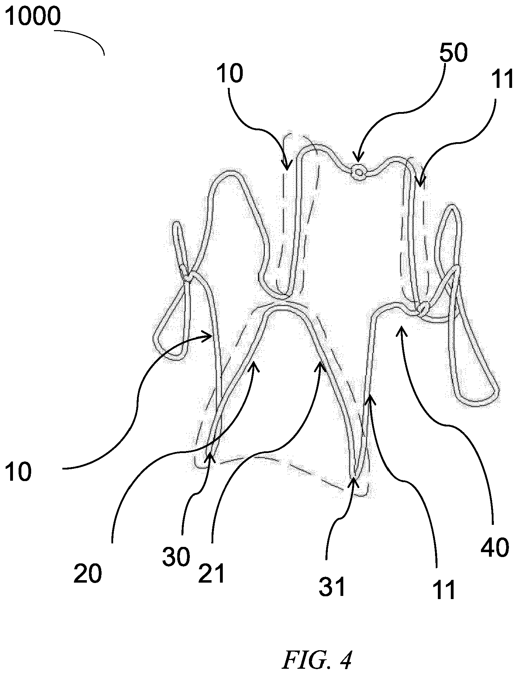

FIG. 4 shows a schematic structure diagram of one of anchoring devices according to the present application. The entire anchoring device 1000 is a closed loop and is formed by bending and winding a memory metal wire, which is preferably made of a nickel-titanium alloy, with a diameter ranging from 0.2 mm to 2 mm. The anchoring device comprises connecting portions and clamping portions. The clamping portions each are a main part for applying a clamping force to an artificial heart valve prosthesis, and the number of the clamping portions corresponds to the number of heart valves. The connecting portions are configured to connect the clamping portions to form a closed loop. In this embodiment, there are three clamping portions and three connecting portions, and the clamping portions and the connecting portions are alternately arranged, that is, two clamping portions are connected by one connecting portion. In this embodiment, the clamping portions each comprise two symmetrical clamping ends, wherein one clamping end comprises a first supporting section 10, a first expansion section 30, and a second supporting section 20. The first expansion section 30 forms a radial opening angle to form pressures towards the blood vessel wall side and the heart valve side. The first supporting section 10 and the second supporting section 20 are curved sections extending from the first expansion section 30 in a vertical direction (an axial direction of the heart valve stent), and are configured to form vertical support for the blood vessel wall or the heart valve and transmit an expansion force released by the first expansion section to the blood vessel wall and the heart valve (or the heart valve prosthesis). The first supporting section 10, the second supporting section 20 and the first expansion section 30 are actually a continuous and integrated memory metal curved section. The second clamping end that is symmetrical with the first clamping end in shape comprises a sixth supporting section 21, a fourth expansion section 31, and a seventh supporting section 11. A continuous and integrated memory metal curved section is also provided between the first clamping end and the second clamping end. A transition connecting section between the second supporting section 20 and the sixth supporting section 21 is a U-shaped or V-shaped curved section. Each connecting portion 40 is a curved section arranged in the circumferential direction, and the curve thereof may comprise an undulating wave section in arrangement. In an embodiment, the connecting portion 40 is further provided with a coupling port 50 for coupling to an application apparatus of the anchoring device.

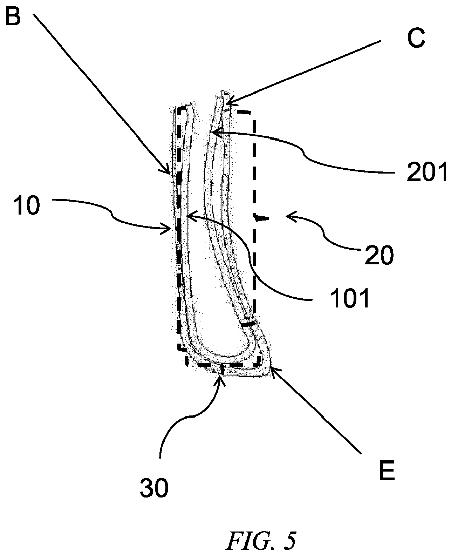

FIG. 5 shows a schematic side view of a partial structure of the embodiment shown in FIG. 4, wherein the first supporting section 10 is located on a side of the heart valve B, the second supporting section 20 is located on a side of the blood vessel wall C, and the first expansion section 30 is located at the bottom of the aortic sinus E. In an embodiment, the first supporting section 10 and the heart valve B form a continuous contact section, and the contact section is a first contact section 101 shown in the figure. In an embodiment, the first supporting section 10 can also form multipoint contact with the heart valve B. In an embodiment, an upper end 201 and a lower end of the second supporting section 20 form contact with the blood vessel wall C, and a curved section protruding in the direction of the center of the blood vessel is formed between the upper end 201 and the lower end, so that the upper end 201 forms a stronger supporting force for the blood vessel wall C.

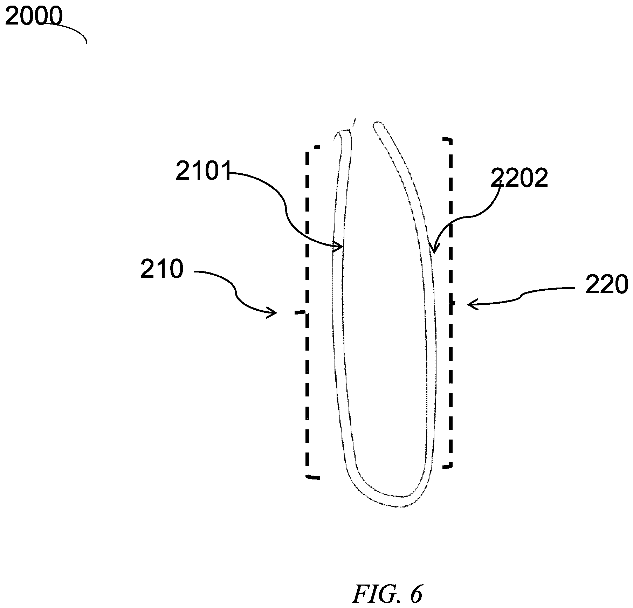

FIG. 6 shows a schematic structure diagram of a clamping end of an anchoring device 2000 in another embodiment. An eighth supporting section 210 is located on the heart valve side, and forms a connecting contact section with the heart valve, that is, a second contact section 2101 therein. A ninth supporting section 220 is located on the blood vessel wall side, and also forms a connecting contact section with the blood vessel wall, that is, a third contact section 2202 in the figure. The clamping end is integrally formed into a slightly-closed U shape in the radial direction.

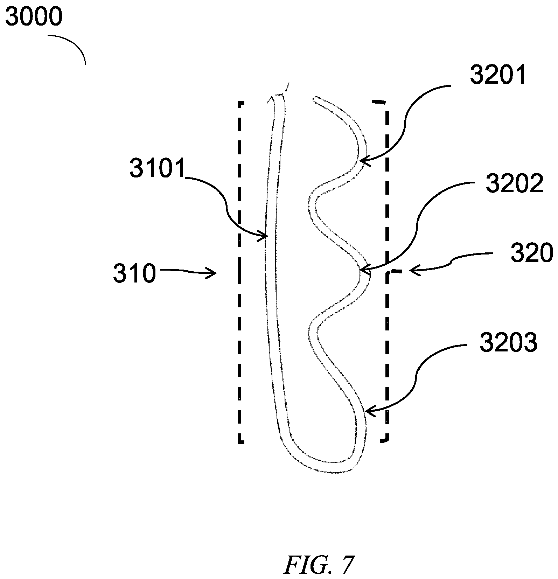

FIG. 7 shows a schematic structure diagram of a clamping end of an anchoring device 3000 in another embodiment. A tenth supporting section 310 is located on the heart valve side, and forms continuous contact with the heart valve, that is, a fourth contact section 3101 therein. An eleventh supporting section 320 is located on the blood vessel wall side, and forms multipoint contact with the blood vessel wall, that is, a second contact point 3201, a third contact point 3202 and a fourth contact point 3203 in the figure. Vertexes of the second contact point 3201, the third contact point 3202 and the fourth contact point 3203 may be at the same height or different heights, and curves therein are connected to make curves where the second contact point 3201, the third contact point 3202 and the fourth contact point 3203 are located form a wave shape, so as to form a stronger supporting force with the blood vessel wall and be not likely to slide out of the anchoring position.

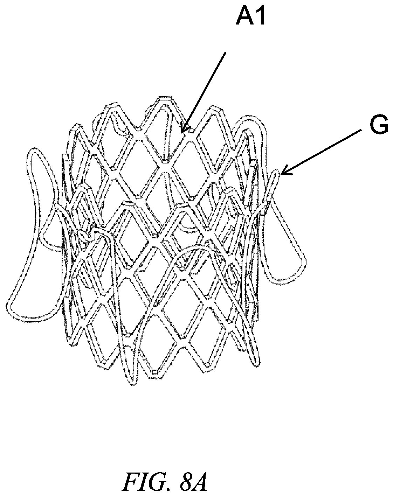

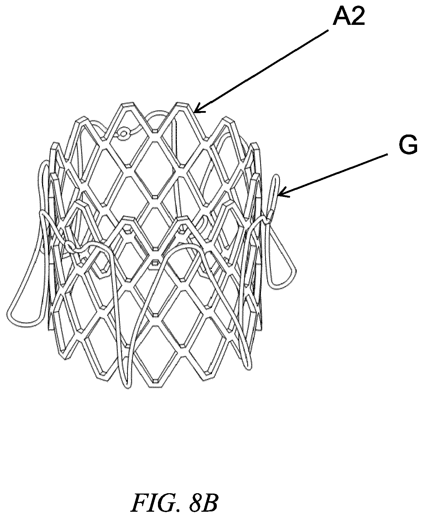

FIGS. 8A-8B show schematic state diagrams of an anchoring device G according to one of embodiments of the present application after being anchored with two heart valve prostheses with different specifications. It can be seen that FIG. 8A shows a smaller heart valve prosthesis Al, and a clamping end of the anchoring device G has a better radial deformation effect, such that the smaller heart valve prosthesis A1 can be properly anchored; and FIG. 8B shows a larger heart valve prosthesis A2, and a clamping end of the anchoring device G has a smaller radial deformation, but the heart valve prosthesis A2 can also be properly fixed because of a larger circumferential dimension of the prosthesis. Therefore, the anchoring device G according to the present application can adapt to heart valve prostheses with different specifications and sizes, and can achieve good anchoring effects.

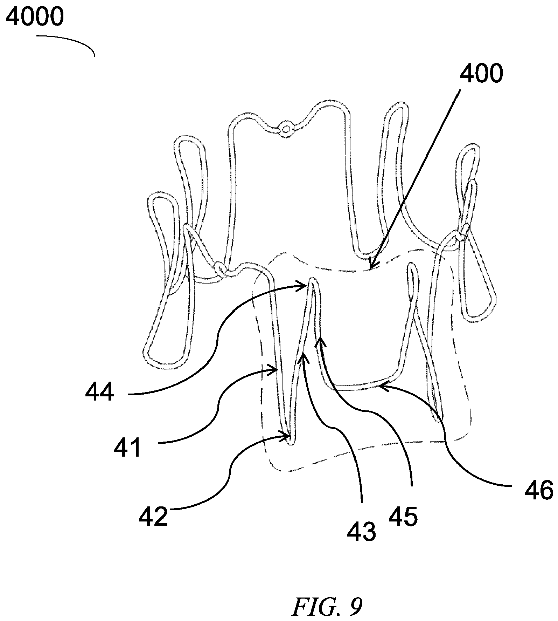

FIG. 9 shows a schematic structure diagram of another anchoring device 4000 according to the present application. The anchoring device comprises three clamping portions. Each clamping portion 400 shown in the figure comprises two symmetrical clamping ends, and each clamping end further comprises three supporting sections and two expansion sections that are in continuous and transition connection with each other, that is, a third supporting section 45, a fourth supporting section 41, a fifth supporting section 43, a third expansion section 42, and a fourth expansion section 44 that are shown in the figure. The third expansion section 42 and the fourth expansion section 44 each comprise a radial opening angle, and are arranged opposite to each other one above the other and are connected by the fifth supporting section 43. The fourth supporting section 41 connects the third expansion section 42 to the connecting portion. The third supporting section 45 is located on the other side of the fourth expansion section 44 and is configured to connect the other clamping end or connecting portion. In an embodiment, the third supporting section 45 is connected to the other clamping end by a connecting section 46. The two expansion sections are arranged in the clamping end to make the clamping end form an upper radial expansion force and a lower radial expansion force, so that the anchoring device will generate a greater radial force compared with the embodiment shown in FIG. 4. The three supporting sections connected to the two expansion sections will apply radial expansion forces and form longitudinal support on the abutted blood vessel wall or heart valve (or the heart valve prosthesis). Where the overall dimension of the anchoring device is reduced, the same or greater radial force can be generated, which saves on a space of a conveying device and reduces a wound size of a patient. In the case of the same overall dimension and the same space of the conveying device, the radial force range is larger, and an artificial heart valve prosthesis with a larger specification can be accommodated.

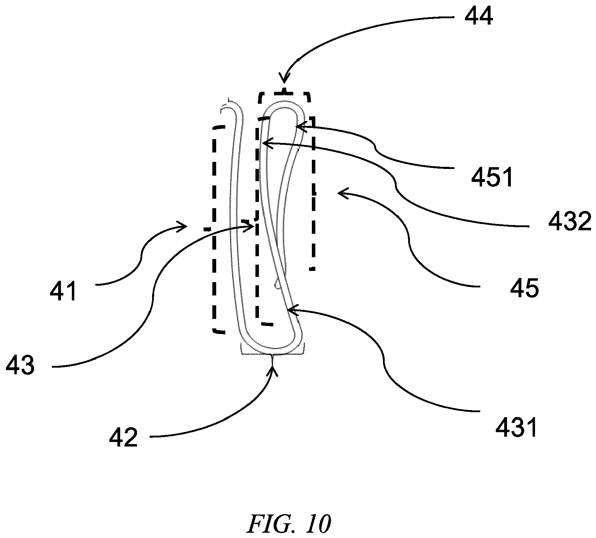

FIG. 10 is a schematic side view of a clamping end of the anchoring device shown in FIG. 9. The fourth supporting section 41 is located on the heart valve side, and forms continuous contact for at least one section with the heart valve. The fifth supporting section 43 is located between the heart valve and the blood vessel wall, and two end portions of the fifth supporting section respectively abut or protrude in different directions. As shown in the figure, a top end portion 432 protrudes towards the heart valve side, and a bottom end portion 431 protrudes towards the blood vessel wall side and abuts against the blood vessel wall. The fourth expansion section 44 is located above opposite to the third expansion section, and the third supporting section 45 extends from the other side of the fourth expansion section, is located on the blood vessel wall side and abuts against the blood vessel wall at at least one point, that is, a fifth contact point 451 shown in the figure. In an embodiment, the third supporting section 45 may also form continuous-section contact with the blood vessel wall.

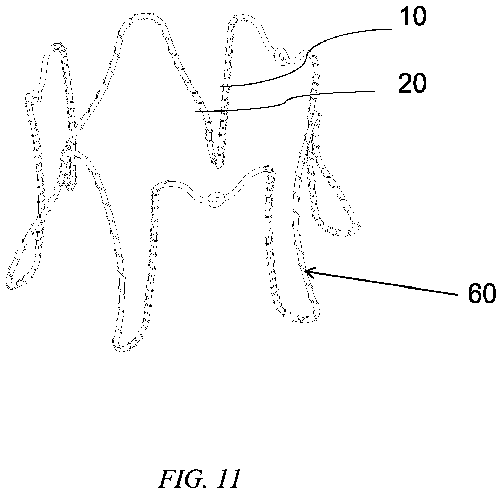

FIG. 11 shows an anchoring device according to another embodiment. A basic structure of a clamping portion of the anchoring device is the same as the structure shown in FIGS. 4 and 5. However, in this embodiment, a second memory metal wire 60 is further wound around the clamping portion, and the second memory metal wire may be wound around a metal wire of the entire anchoring device, or only around the clamping portion, or only around a part of the clamping portion. In the figure, the second memory metal wire is wound around the first supporting section 10, the second supporting section 20, and the expansion section, with the purpose of enhancing the expansion or supporting strength of the anchoring device and enhancing the contact with the blood vessel wall and the heart valve wall.

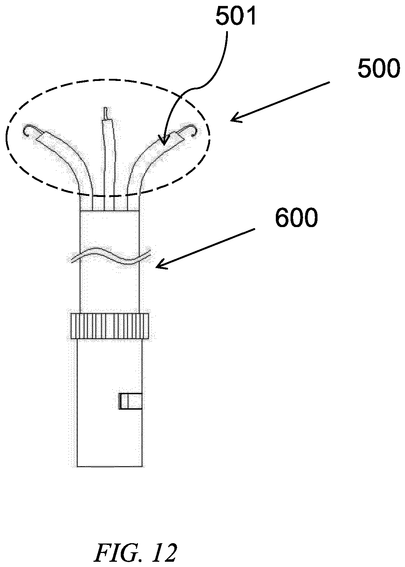

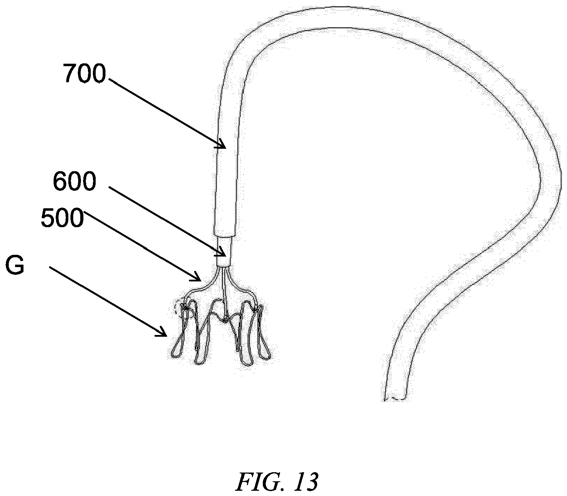

FIGS. 12 and 13 show an auxiliary tool for applying an anchoring device between a heart valve and a blood vessel wall. The auxiliary tool comprises a claw-shaped fitting 500, which comprises a root and pawls 501 connected to the root. The pawls each are a finger and an end thereof is provided with a hook. In an embodiment, there are three pawls. In an embodiment, there are two pawls. The auxiliary tool further comprises a manipulator 600, and an end portion of the manipulator 600 is connected to the root of the claw-shaped fitting. In one of embodiments, the end portion of the manipulator 600 is connected to the claw-shaped fitting by means of a thread. In one of embodiments, the end portion of the manipulator 600 is in nested connection with the claw-shaped fitting by means of a fastener. The manipulator 600 generally comprises a long and narrow metal guide wire, and the manipulator 600 enters a surgical site through a sheath 700. In an interventional surgery, after the anchoring device G and the claw-shaped fitting 500 reach the surgical site by means of the manipulator 600 and the sheath 700, the anchoring device G is enabled to be disengaged from the claw-shaped fitting 500, and the anchoring device G forms the state shown in FIG. 8A or 8B or the position state shown in FIG. 2 after self-expanding.

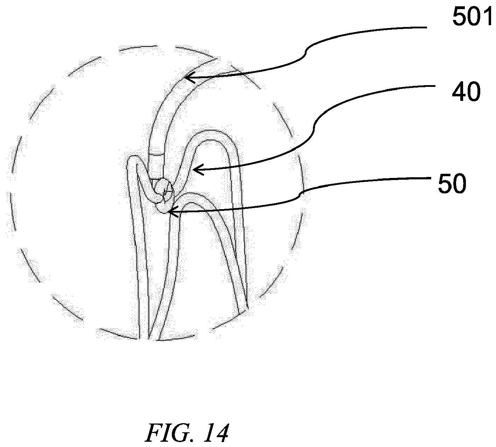

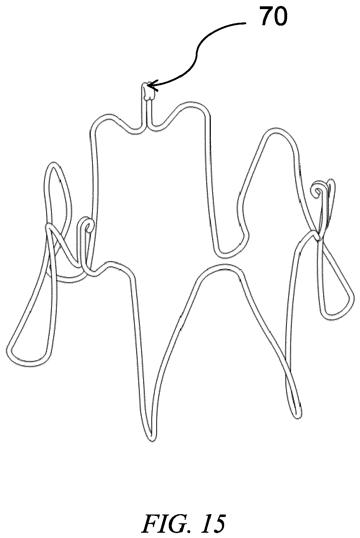

FIGS. 14 and 15 show two methods in which the anchoring device is coupled to the claw-shaped fitting. In FIG. 14, the coupling port 50 is a ring, and is arranged at an approximate middle position of the connecting portion 40, and the hook on the end portion of the pawl 501 is in a coupled state when engaged with the ring. In FIG. 15, the coupling port is a hook-shaped port 70, and the hook on the end portion of the pawl 501 is in a coupled state when engaged with the hook-shaped port 70. In some embodiments, the coupling port may have other shapes such as a diamond and an ellipse.

In an embodiment, the anchoring device is pre-arranged in the claw-shaped fitting and a manipulator tube before leaving the factory, coupling ports of the anchoring device are respectively mounted on the pawls of the claw-shaped fitting, then a release buckle is pushed to push the manipulator to a locking position, and the coupling ports are clamped by the manipulator and the pawls. During surgery, the sheath is first sent to a predetermined release position, and then a combination of the anchoring device and the claw-shaped fitting is inserted into the sheath by means of the manipulator; after the manipulator is pushed to reach the predetermined release position, the sheath is withdrawn on the basis of applying a certain acting force on the manipulator to keep the manipulator motionless, so that the claw-shaped fitting is exposed, and then a knob is turned to push the manipulator forward, to push the anchoring device to the predetermined position; and then the release buckle is pulled, such that the release buckle drives the pawls to move to a proximal end, thereby making the pawls drop from the coupling port to complete the deployment of the anchoring device.

The present application is use for clamping an artificial valve implanted in a transcatheter manner, so that the artificial valve can be suitable for heart valve regurgitation. When in use, the anchoring device according to the present application is first placed in the aortic root in a transcatheter manner, the device is placed in the aortic sinus, and the proximal end of the device is closely attached to the bottom of the aortic sinus. Then the artificial heart valve prosthesis is implanted by transcatheter aortic valve implantation (TAVI), and after the artificial heart valve prosthesis is expanded, a combination of the artificial heart valve prosthesis and the anchoring device is formed. A circumferential force is applied towards the center of the blood vessel from a near-valve side of the anchoring device according to the present application, and a circumferential force towards the blood vessel wall side is applied to the aortic root from a near-wall side. There are supporting forces for the artificial heart valve prosthesis on the middle portion of the combination and the blood vessel wall, so that stress compensation can be performed between the near-valve side and the near-wall side of the anchoring device according to different dimensions and specifications of artificial heart valve prostheses, to achieve effects of self-adapting to artificial heart valve prostheses with different dimensions and specifications. By means of the interacting forces, the artificial heart valve prosthesis is fixed to the position of the aortic valve, which benefits patients with aortic valve regurgitation and aortic valve insufficiency.

The exemplary embodiments in this description provide methods by way of example only, and examples of one method are not intended to limit examples of another method. A device/method illustrated in a figure may be added to or exchanged with devices/methods in other figures. Furthermore, specific digital data values (for example, specific numbers, quantities, categories, etc.) or other specific information are used merely to describe the exemplary embodiments and are not intended to limit the exemplary embodiments with such specific information.

## Claims

1. An artificial heart valve prosthesis anchoring device, comprising:
a memory metal wire, wherein the memory metal wire is bent and encircled to form a closed loop configured to anchor an artificial heart valve stent, and the closed loop comprises:
at least two clamping portions configured to form an expansion force in a radial direction and a restraint force in a circumferential direction to anchor the anchoring device between a heart valve and an adjacent blood vessel wall; and
connecting portions that quantitatively correspond to the clamping portions, are alternately arranged with the clamping portions, and are used for transition and connection of adjacent clamping portions, wherein
a curve of each clamping portion comprises at least one clamping end, and in a mounted state, each clamping end comprises:
a first expansion section with a radial opening angle, and
a first supporting section and a second supporting section that extend from two ends of the first expansion section, wherein at least a part of the first supporting section and the second supporting section abuts against a blood vessel wall and a valve wall, and the first expansion section abuts against a connection part between the blood vessel wall and the valve wall.

2. The artificial heart valve prosthesis anchoring device according to claim 1, wherein each of the connecting portions is a curved section between two adjacent clamping portions, and a middle portion of the connecting portion is provided with a coupling port configured to be coupled to or released from an instrument for operating the anchoring device.

3. The artificial heart valve prosthesis anchoring device according to claim 1, wherein when the anchoring device is applied to an anchoring position, the second supporting section comprises at least a part in continuous contact with the blood vessel wall.

4. The artificial heart valve prosthesis anchoring device according to claim 1, wherein when the anchoring device is applied to an anchoring position, the second supporting section forms multipoint contact with the blood vessel wall.

5. The artificial heart valve prosthesis anchoring device according to claim 1, wherein when the anchoring device is applied to an anchoring position, the first supporting section comprises at least a part in continuous contact with the heart valve.

6. The artificial heart valve prosthesis anchoring device according to claim 1, wherein when the anchoring device is applied to an anchoring position, the first supporting section is in contact with the heart valve to form multipoint contact.

7. The artificial heart valve prosthesis anchoring device according to claim 1, wherein the clamping end further comprises a second expansion section with a second radial opening angle, the second expansion section is connected to the first expansion section by the second supporting section, and a third supporting section extends from the other end of the second expansion section.

8. The artificial heart valve prosthesis anchoring device according to claim 7, wherein the second expansion section and the first expansion section are rotationally symmetric.

9. The artificial heart valve prosthesis anchoring device according to claim 8, wherein the first expansion section and/or the second expansion section are/is generally V-shaped or U-shaped.

10. The artificial heart valve prosthesis anchoring device according to claim 7, wherein the second supporting section connecting the first expansion section to the second expansion section forms contact with the blood vessel wall and/or the heart valve.

11. The artificial heart valve prosthesis anchoring device according to claim 7, wherein when the anchoring device is applied to an anchoring position, the third supporting section forms continuous-part contact or multipoint contact with the blood vessel wall.

12. The artificial heart valve prosthesis anchoring device according to any one of claims 1 to 11, wherein the clamping portion comprises two substantially symmetrical clamping ends.

13. The artificial heart valve prosthesis anchoring device according to claim 11, wherein the two clamping ends are connected by a fourth supporting section, and the fourth supporting section is a curved section arranged in the circumferential direction.

14. The artificial heart valve prosthesis anchoring device according to claims 1 to 11, wherein a second memory metal wire is further wound around the anchoring device, and the second memory metal wire is wound at least around a part of the clamping portion.

15. The artificial heart valve prosthesis anchoring device according to claim 2, wherein the coupling port is ring-shaped or hook-shaped.

16. An artificial aortic valve anchoring kit, comprising:
an anchoring device according to any one of claims 1 to 15; and
a claw-shaped fitting comprising a root and at least two pawls connected to the root, wherein
when the coupling port of the anchoring device is coupled to the pawl, the anchoring device is in a to-be-released state; and when the coupling port of the anchoring device is uncoupled from the pawl, the anchoring device is placed at a target valve position to be self-expanded to a set state.

17. The artificial aortic valve anchoring kit according to claim 16, further comprising a manipulator, wherein an end portion of the manipulator is connected to the claw-shaped fitting, and the manipulator is configured to push the anchoring device and the claw-shaped fitting towards a distal port of a catheter by means of the catheter until the anchoring device and the claw-shaped fitting are pushed out of the distal port of the catheter.

18. An artificial aortic valve prosthesis anchoring device, comprising:
a memory metal wire, wherein the memory metal wire is bent and encircled to form a closed loop configured to anchor an artificial heart valve stent, and the closed loop comprises:
at least two clamping portions configured to form an expansion force in a radial direction and a restraint force in a circumferential direction to anchor the anchoring device between a blood vessel wall and a heart valve; and
connecting portions that quantitatively correspond to the clamping portions, are alternately arranged with the clamping portions, and are used for transition and connection of adjacent clamping portions, wherein
a curve of each clamping portion comprises two clamping ends adjacent and connected to each other, and in a mounted state, each clamping end comprises:
a first expansion section with a radial opening angle, the first expansion section being generally V-shaped or U-shaped; and
a first supporting section and a second supporting section that extend from two ends of the first expansion section;
when the anchoring device is applied to an anchoring position, the first expansion section and the extended first supporting section comprise at least a part in continuous contact with the heart valve or form multipoint contact with the heart valve; and the first expansion section and the extended second supporting section comprise at least a part in continuous contact with the blood vessel wall or form multipoint contact with the blood vessel wall.

19. The artificial aortic valve prosthesis anchoring device according to claim 18, wherein the clamping end further comprises a second expansion section with a second radial opening angle, the second expansion section is connected to the first expansion section by the second supporting section, and a third supporting section extends from the other end of the second expansion section.

20. The artificial aortic valve prosthesis anchoring device according to claim 18, wherein a second memory metal wire is further wound around the anchoring device, and the second memory metal wire is wound at least around a part of the clamping portion.

21. A using method for an artificial aortic valve prosthesis anchoring device, comprising:
self-expanding, after an anchoring device according to claims 1 to 15 is placed at an anchoring position by means of a catheter, the anchoring device to release and form an expansion force towards an artificial aortic valve stent and a blood vessel wall, and subjecting the anchoring device to an opposite acting force from the artificial aortic valve stent and the blood vessel wall, thereby forming a force balance to anchor the artificial aortic valve stent.

22. The using method for an artificial aortic valve prosthesis anchoring device according to claim 21, wherein the anchoring device is applied to the anchoring position by means of a manipulator and a claw-shaped fitting via an interventional catheter.

23. The using method for an artificial aortic valve prosthesis anchoring device according to claim 22, wherein the anchoring position is an aortic sinus.
